# EUROPÄISCHE PATENTSCHRIFT

(11) **EP 0 661 984 B1**
(45) Veröffentlichungstag und Bekanntmachung des Hinweises auf die Patenterteilung: **03.04.1996**
(21) Anmeldenummer: 93919312.4
(22) Anmeldetag: 09.09.1993
(51) Int. Cl.: A61K 35/78

(54) **VERWENDUNG VON BÄRLAUCH ZUR THERAPIE ODER PROPHYLAXE VON HERZRHYTHMUSSTÖRUNGEN**
USE OF WILD GARLIC FOR THE THERAPY OR PREVENTION OF HEART RHYTHM DISTURBANCES
UTILISATION D'AIL DES OURS DANS LE TRAITEMENT OU LA PROPHYLAXIE DE TROUBLES DU RYTHME CARDIAQUE

(30) Priorität: 09.09.1992 DE 4230189
(43) Veröffentlichungstag der Anmeldung: 12.07.1995
(73) Patentinhaber: PANDALIS, Georgios, D-49219 Glandorf (DE)
(72) Erfinder: PANDALIS, Georgios, D-49219 Glandorf (DE); RIETZ, Bettina, D-72108 Rottenburg am Neckar (DE); JACOB, Ruthard, D-72076 Tübingen (DE)
(74) Vertreter: Grünecker, Kinkeldey, Stockmair & Schwanhäusser Anwaltssozietät
(86) Internationale Anmeldenummer: EP9302443
(87) Internationale Veröffentlichungsnummer: WO9405307

(56) Entgegenhaltungen:
- BIOLOGICAL ABSTRACTS, xol. 95, no. 12 15. Juni 1993, Philadelphia, PA, US; abstract no. 134728, RIETZ B. ET AL. 'CARDIOPROTECTIVE ACTIONS OF WILD GARLIC (ALLIUM URSINUM) IN ISCHEMIA AND REPERFUSION'
- THE AMERICAN JOURNAL OF CHINESE MEDICINE Bd. 7, Nr. 3 , 1979 Seiten 197 - 236 V. PETKOV 'PLANTS WITH HYPOTENSIVE, ANTIATHEROMATOUS AND CORONARODILATATING ACTION'
- PLANTA MEDICA Bd. 58, Nr. 1 , Februar 1992 Seiten 1 - 7 A. SENDL ET AL. 'COMPARATIVE PHARMACOLOGICAL INVESTIGATIONS OF ALLIUM URSINUM AND ALLIUM SATIVUM'
- THERAPIEWOCHE Bd. 40, Nr. 46 , 12. November 1990 Seite 3349 HANS-JÜRGEN RICHTER 'PFLÜCKEN SIE SICH EINEN ACE-HEMMER !'
- BIOLOGICAL ABSTRACTS, vol. 95, no. 12, 15. Juni 1993, Philadelphia, PA, US, abstract no. 134728; RIETZ B. ET AL.: 'CARDIOPROTECTIVE ACTIONS OF WILD GARLIC (ALLIUM URSINUM) IN ISCHEMIA AND REPERFUSION'

## Beschreibung

Die Erfindung betrifft die Verwendung von Allium ursinum L. (Bärlauch).

Allium ursinum L. (Bärlauch, Bärenlauch, wilder Knoblauch) gehört, wie der Knoblauch, zur Familie der Liliaceae. Die Pflanze ist u.a. in fast ganz Europa beheimatet und wird auch als Heil-, Gewürz- und Gemüsepflanze kultiviert. Für Arzneimittel werden die Bärlauchzwiebel (Allii ursini bulbus), das Bärlauchkraut (Allii ursini herba) sowie die ganze, frische zur Blütezeit gesammelte Pflanze für homöopathische Zwecke verwendet. Die Inhaltsstoffe der Zwiebel und des Krautes sind als identisch anzusehen. (s. Hagers Handbuch der Pharmazeutischen Praxis, 4. Band, 5. Auflage, Springer-Verlag, im Druck).

In der Bärlauchzwiebel lassen sich abhängig von Extraktionsmedium und -verfahren unterschiedliche Muster an genuinen und nicht genuin enthaltenen Inhaltsstoffen nachweisen. Dazu gehören Cysteinsulfoxide, wie S-Methyl-L-(+)-cysteinsulfoxid und S-Allyl-L-(+)-cysteinsulfoxid (Alliin) und Thiosulfinate, die durch Fermentation aus den Cysteinsulfoxiden gebildet werden. Beispiele für die Thiosulfinate sind Allylmethylthiosulfinat bzw. Methylallylthiosulfinat, Diallylthiosulfinat (Allicin) und Dimethylthiosulfinat. Weitere, nicht genuin in der Bärlauchzwiebel enthaltene Stoffe sind Dithiine, wie Vinyldithiine, Ajoen (4,5,9-Trithiododeca-1,6,11-trien-9-oxid), das durch Selbstkondensation von Allicin entsteht, sowie Ajoenhomologe. Ferner werden als wasserdampfflüchtige Bestandteile (12 % bezogen auf die frische Bärlauchzwiebel) Methylallyltrisulfid, das den Hauptbestandteil des Wasserdampfdestillats bildet, sowie geringe Mengen an Diallyldisulfid und Diallyltrisulfid erhalten.

Diese Stoffe sind ebenfalls nicht genuin in der Bärlauchzwiebel enthalten. Weitere, genuin in der Bärlauchzwiebel enthaltene Stoffe sind beispielsweise die freien Aminosäuren L-Arginin, L-Asparaginsäure, L-Glutaminsäure, L-Asparagin, L-Glutamin, L-Glycin, L-Threonin und L-Alanin.

Die frische Zwiebel enthält nach der Fermentation etwa 0,05 bis 0,12 % Allicin.

Untersuchte Wirkungen der Bärlauchzwiebel sind eine Lipoxygenase- und Cyclooxygenasehemmung, sowie eine Hemmung im PAF-Thrombozytenaggregationstest.

Frische Bärlauchblätter enthalten nach der Fermentation etwa 0,005 % Allicin, getrocknete etwa 0,07 %. Ferner enthalten frische Bärlauchblätter etwa 0,007 % wasserdampfflüchtige Bestandteile, die den wasserdampfflüchtigen Bestandteilen der Bärlauchzwiebel entsprechen.

Volkstümlich werden Bärlauchblätter und -zwiebeln, ähnlich wie Allium sativum (Knoblauch), bei Magen-Darm-Störungen, Gärungsdysepsien, Flatuleszenz, gegen Bluthochdruck und Arteriosklerose eingesetzt. Äußerlich finden sie Anwendung bei Hautausschlägen. Die Wirksamkeit bei den genannten Anwendungsgebieten ist nicht belegt.

Die Urtinktur aus ganzen, frischen, zu Beginn der Blütezeit gesammelten Bärlauchpflanzen HAB1 ist eine goldgelbe Flüssigkeit mit Geruch und Geschmack nach Knoblauch HAB1. Die Anwendungsgebiete entsprechen dem homöopathischen Arzneimittelbild. Dazu gehört beispielsweise Verdauungsschwäche.

Ein Einsatz von Bärlauch in der Schulmedizin erfolgt nicht.

Wenn das Herz mit Blut und somit mit Sauerstoff unterversorgt wird, wie es beispielsweise bei einem Herzinfarkt der Fall ist, treten Herzrhythmusstörungen als häufigste Komplikation auf und erschweren die Behandlung des Patienten.

Rhythmusstörungen des Herzens sind Abweichungen der zeitlichen Folge und/oder Regelmäßigkeit der Herzaktionen von der normalen Herzfrequenz, ausgelöst durch pathologische Veränderungen des Herzmuskels oder vegetative Einflüsse. Ihrem Wesen nach handelt es sich um Störungen der Erregungsbildung (normo- oder heterotop) sowie der Erregungsleitung. Klinisch besonders bedeutsam sind die bei beeinträchtigter Koronarperfusion auftretenden Rhythmusstörungen, die zu plötzlichem Herztod führen können (Pschyrembel, Klinisches Wörterbuch, 255. Auflage, Verlag Walter de Gruyter, Berlin, New York 1986).

Rhythmusstörungen bei vermindert perfundiertem Herz werden beispielsweise mit Beta-Rezeptorenblockern oder Calciumantagonisten behandelt.

Aufgabe der vorliegenden Erfindung ist es, ein Mittel auf natürlicher Basis zur Prophylaxe oder Behandlung von Herzrhythmusstörungen zur Verfügung zu stellen.

Diese Aufgabe wird erfindungsgemäß gelöst durch die Verwendung von Allium ursinum L. (Bärlauch) zur Herstellung eines Medikaments zur Prophylaxe oder Behandlung von Herzrhythmusstörungen.

Die Verwendung von Bärlauch ist besonders geeignet zur Prophylaxe oder Behandlung, insbesondere der unterstützenden Behandlung, von Herzrhythmusstörungen bei vorgeschädigtem, d.h. vermindert perfundiertem Herz.

Insbesondere kann Bärlauch eingesetzt werden, um einen Rückgang oder einen späteren Eintritt primärer ventrikulärer Tachykardien und ventrikulärer Fibrillationen bei einem, beispielsweise durch Minderdurchblutung im Rahmen einer koronaren Herzkrankheit (KHK), vorgeschädigten, d.h. vermindert perfundierten Herz, zu erreichen.

Zur Erzielung der antiarrhythmischen Wirkung von Bärlauch kann die ganze Pflanze in frischem oder getrocknetem Zustand, sowie einzelne frische oder getrocknete Pflanzenteile, wie die Bärlauchzwiebel oder das Bärlauchkraut, eingesetzt werden. Weiterhin sind auch verschiedene Extrakte der Bärlauchzwiebel und/oder des -krauts, die durch Wasserdampfdestillation erhaltenen Fraktionen und die Urtinktur sowie Mischungen daraus wirksam.

Erfindungsgemäß wird Bärlauch bevorzugt in Form üblicher pharmazeutischer Zubereitungen, wie Lösungen, Konzentraten, Tabletten oder Kapseln oral verabreicht. Nach erfolgter klinischer Prüfung wird die benötigte Dosierung der Wirkstoffe in Abhängigkeit von üblichen Faktoren, wie der Natur und Schwere der Erkrankung und dem Körpergewicht des Patienten, vom Arzt festgelegt.

Das folgende Beispiel zeigt die antiarrhythmische Wirkung von Bärlauch bei Wistar-Ratten.

### Beispiel

Zwei Monate alte Wistar-Ratten wurden über einen Zeitraum von acht Wochen mit einer speziellen Diät ernährt. Es wurde mit zwei verschiedenen Gruppen gearbeitet:
a) Die Kontrollgruppe erhielt die Standard-Diät.
b) Die Testgruppe erhielt einen 2 %-igen Zusatz von Bärlauch (gepulverte, getrocknete Blätter) zu der Standard-Diät.

Nach dem Fütterungszeitraum wurden die Tiere abgetötet und die Herzen sofort entnommen. Anschließend wurde sofort mit einer rückläufigen Durchspülung der Aorta, wie von Langendorff beschrieben, mit einer Krebs-Henseleit-Lösung begonnen. Die Temperatur betrug 37 ^{o}C, der Druck 5,2 kPa. Nach einer Zeit zur Einstellung des Gleichgewichts wurde die linke koronare Arterie verschlossen.

Anschließend wurde mit zwei verschiedenen Verfabren gearbeitet:
1) Occlusion
   Der Arterienverschluß wurde 20 Min. aufrechterhalten. Dieser Verschluß bewirkt eine akute Ischämie des Herzens und imitiert einen Herzinfarkt.
2) Reperfusion
   Die Arterie wurde nach 8 Min. wieder geöffnet. Die Reperfusion hat drei praktische Bedeutungen:
   1. bei der Wiederherstellung des vorher durch Ischämie stehengebliebenen Herzens, wie bei Herzoperationen;
   2. bei myocardialer Infarktbildung mit akuter Revascularisation durch Thrombolyse;
   3. bei temporären schweren Koronarspasmen.

### EKG

Zur Erfassung der Rhythmusstörungen wurden direkte Ableitungen des Elektrokardiogramms am Herzen vorgenommen. Es wurden folgende Parameter bestimmt:
- Die Dauer des Sinusrhythmus;
- Wann und wie lange ventriculare Tachycardien auftreten;
- Ab wann Flimmern auftritt

### Ischämische Zone

Die ischämische Zone kann man mit einem nekrotischen Bereich im Herzmuskel vergleichen, der durch Sauerstoffmangel (Anoxie), hervorgerufen durch eine Unterbrechung der Blutzufuhr zu diesem Bereich, entstanden ist.

Nach der Verschlußzeit von 20 Min. wurde ein Bolus aus Malachitgrün-Farbstoff in die Aorta gespritzt, um den durchspülten grünen Bereich vom nicht durchspülten roten Bereich über eine Anfärbung zu unterscheiden. Der nicht durchspülte (rote) Bereich wurde herausgeschnitten, trockengetupft und gewogen, um die Größe der Risikozone prozentual zum gesamten Herzgewicht zu bestimmen.

### Ergebnisse

- zu 1) -: Bärlauch steigerte die Gesamtdauer des Sinusrhythmus in der Verschlußphase von 3,4 Min. (Kontrollgruppe) auf 7,2 Min. (Testgruppe).
- Außerdem trat eine signifikante Verringerung des Auftretens von Flimmern auf: Bei 88 % der Tiere in der Kontrollgruppe wurde Flimmern hervorgerufen, das im Durchschnitt nach 11,5 Min. begann. In der Testgruppe trat Flimmern nur bei 20 % der Ratten auf, das durchschnittlich nach 18 Min. begann.
- Der undurchspülte ischämische Bereich der Herzen war in der Testguppe kleiner als in der Kontrollgruppe: In der Testgruppe wurden 33, 6 Gew.-% gemessen, in der Kontrollgruppe 40,9 Gew.-%.
- zu 2) -: Alle Kontrolltiere zeigten ventriculare Fibrillation (Flimmern), wogegen nur bei 70 % der Testtiere Flimmern auftrat.

## Patentansprüche

1. Verwendung von Allium ursinum L. (Bärlauch) zur Herstellung eines Medikaments zur Behandlung oder Prophylaxe von Herzrhythmusstörungen.

2. Verwendung nach Anspruch 1 bei vorgeschädigtem, d.h. vermindert perfundiertem Herz.

3. Verwendung nach Anspruch 1 oder 2, **dadurch gekennzeichnet**, daß der Bärlauch in oraler Form verabreicht wird.

## Claims

1. Use of Allium ursinum L. (ramsons, wild garlic) for the preparation of a medicine for the treatment or prophylaxis of cardiac arrhythmia.

2. Use according to claim 1 in the previously impaired, that is, restrictedly perfused heart.

3. Use according to claim 1 or 2, characterised in that the wild garlic is administered in oral form.

## Revendications

1. Utilisation d'allium ursinum L (ail d'ours) pour ta préparation d'un médicament pour le traitement ou la prophylaxie de troubles du rythme cardiaque.

2. Utilisation suivant la revendication 1 sur un coeur atteint, c'est-à-dire moins irrigué.

3. Utilisation suivant l'une des revendications 1 ou 2, caractérisée en ce que l'ail d'ours est administré par voie orale.
